(19) Europäisches Patentamt
European Patent Office
Office européen des brevets

(11) Publication number: **0 002 792**
**A1**

# (12) EUROPEAN PATENT APPLICATION

(21) Application number: **78101777.7**

(22) Date of filing: **20.12.78**

(51) Int. Cl.²: **C 07 D 209/44,** A 61 K 31/40, C 07 D 405/12

(30) Priority: **03.01.78 CH 26/78**

(43) Date of publication of application: **11.07.79 Bulletin 79/14**

(84) Designated Contracting States: **BE CH DE FR GB IT LU NL SE**

(71) Applicant: **SANDOZ LTD., Lichtstrasse 35, CH-4002 Basel (CH)**

(72) Inventor: **Achini, Roland, Dr., Teichstrasse 104, CH-4106 Therwil (CH)**
Inventor: **Berthold, Richard, Dr., Ahornstrasse 9, CH-4103 Bottmingen (CH)**

## (54) 3-Aminopropoxyaryl derivatives, their preparation and pharmaceutical compositions containing them.

(57) The compounds of the type having an

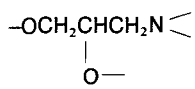

side chain attached through the 1-oxygen atom thereof directly to a heterocyclic nucleus comprising an N-carbonylisoindolinyl radical and wherein the 1-oxygen atom of the side chain is attached directly to the 4 position of the isoindolinyl ring are useful as adrenergic blocking agents.

EP 0 002 792 A1

# 3-AMINOPROPOXYARYL DERIVATIVES, THEIR PREPARATION AND PHARMACEUTICAL COMPOSITIONS CONTAINING THEM

The present invention relates to 3-aminopropoxyaryl derivatives, their preparation and pharmaceutical compositions containing them.

In particular the invention provides adrenergic blocking agents of the type having a $-OCH_2\underset{O-}{CH}CH_2N\langle$ side chain attached through the 1-oxygen atom directly to a heterocyclic nucleus, characterized in that the heterocyclic nucleus comprises an N-carbonylisoindolinyl radical and the 1-oxygen atom of the side chain is attached directly to the 4 position of the isoindolinyl ring, hereinafter referred to as the compounds of the invention.

In accordance with the invention, there are especially provided compounds of formula I

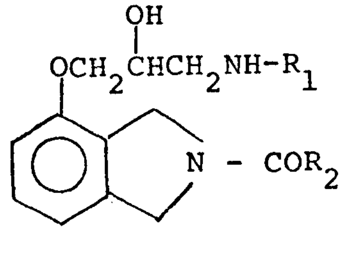

I

wherein

$R_1$ is alkyl of 3 to 7 carbon atoms or a group

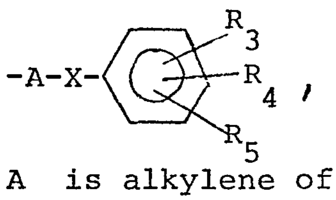, wherein

A is alkylene of 2 to 5 carbon atoms,

X is a bond, an oxygen or a sulfur atom, either

$R_3$ is hydrogen, halogen of atomic number of from 9 to 35, alkyl of 1 to 4 carbon atoms, alkoxy of 1 to 4 carbon atoms or hydroxy, and

$R_4$ is hydrogen and, when $R_3$ is halogen of atomic number of from 9 to 35, $R_4$ additionally may be halogen of atomic number of from 9 to 35 and, when $R_3$ is alkoxy of 1 to 4 carbon atoms, $R_4$ additionally may be alkoxy of 1 to 4 carbon atoms, or

$R_3$ and $R_4$ together are methylenedioxy or ethylenedioxy, in the 2,3 or the 3,4 position,

$R_5$ is hydrogen and, when $R_3$ and $R_4$ both are alkoxy of 1 to 4 carbon atoms, $R_5$ additionally may be alkoxy of 1 to 4 carbon atoms,

with the proviso that X is separated by at least 2 carbon atoms from the nitrogen atom of the 3-aminopropoxy side chain, and

$R_2$ is hydrogen, alkyl of 1 to 4 carbon atoms, alkoxy of 1 to 4 carbon atoms or phenyl unsubstituted or monosubstituted or independently disubstituted by alkyl of 1 to 4 carbon atoms, alkoxy of 1 to 4 carbon atoms, or halogen of atomic number of from 9 to 35,

and physiologically acceptable hydrolyzable derivatives thereof having the hydroxy group in the 2 position of the 3-aminopropoxy side chain in esterified form.

In the compounds of the invention the N-carbonyl radical is preferably a group $-COR_2$ wherein $R_2$ is as defined above.

Physiologically hydrolyzable derivatives are those derivatives which under physiological conditions are split to the corresponding compounds having a hydroxy group in the 2 position of the 3-amino-propoxy side chain.

A group of derivatives in esterified form are e.g. the compounds of formula E

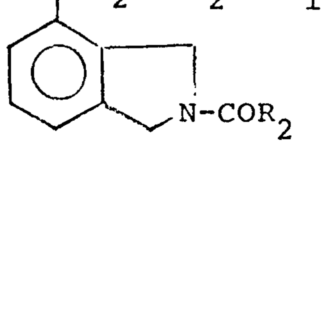

E

wherein

$R_1$ and $R_2$ are as defined above and

$R_e$ is alkyl of 1 to 12 carbon atoms, cycloalkyl of 3 to 7 carbon atoms, phenyl, phenylalkyl of 7 to 12 carbon atoms, phenyl or phenylalkyl of 7 to 12 carbon atoms monosubstituted in the phenyl ring by alkyl of 1 to 4 carbon atoms, or mono- or independently disubstituted in the phenyl ring by halogen of atomic number of from 9 to 35, or mono- or independently di- or independently trisubstituted in the phenyl ring by alkoxy of 1 to 4 carbon atoms.

$R_1$ preferably is a group -A-X- 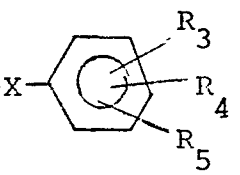 .

X preferably is a bond or oxygen, especially a bond. $R_2$ preferably is hydrogen or alkyl, especially alkyl. $R_3$ preferably is hydrogen, hydroxy, alkoxy or together with $R_4$ methylenedioxy or ethylenedioxy, especially alkoxy. $R_4$ preferably is hydrogen, alkoxy or together with $R_3$ methylenedioxy or ethylenedioxy, especially hydrogen or alkoxy. $R_5$ preferably is hydrogen. $R_e$ preferably is alkyl or phenyl. Alternatively, $R_e$ conveniently is cycloalkyl, substituted phenyl or substituted or unsubstituted phenylalkyl.

Alkyl (except as indicated hereunder for $R_1$ and $R_e$) and/or alkoxy preferably are of 1 or 2, especially of 1 carbon atom . When $R_1$ and/or $R_e$ are alkyl, they

preferably are of 3 to 5 carbon atoms and preferably are branched, especially in the position α to the nitrogen atom to which they are bound. Interesting alkyl groups $R_1$ and/or $R_e$ are e.g. isopropyl, tert-butyl and 3-pentyl, especially tert-butyl. Halogen preferably is bromine or chlorine, especially chlorine. Cycloalkyl preferably is of 5 or 6 carbon atoms.

A and/or the alkylene moiety of $R_e$ when it is phenylalkyl of more than 8 carbon atoms preferably is branched alkylene, especially in the position α to the nitrogen atom or carbonyl moiety to which it is bound, as e.g. in the group $-\overset{\alpha}{C}H(CH_3)-CH_2-$, $-\overset{\alpha}{C}(CH_3)_2-CH_2-$ or $-\overset{\alpha}{C}(CH_3)_2-(CH_2)_2-$. A especially is ethylene. $R_3$ and $R_4$ when bound together preferably are methylenedioxy.

When $R_3$ is not hydrogen, it preferably is in the para position. When $R_4$ is not hydrogen, it preferably is in the meta position. When $R_5$ is not hydrogen, it also preferably is in the meta position. When $R_3$ and $R_4$ together are methylenedioxy or ethylenedioxy, they preferably are in the meta and para positions. When $R_2$ is monosubstituted phenyl and/or when $R_e$ is monosubstituted phenyl or phenylalkyl, the substituent

preferably is in the para position. When $R_2$ is disubstituted phenyl and/or when $R_e$ is di- or trisubstituted phenyl or phenylalkyl, the substituents preferably are in the meta and para positions.

When $R_4$ is not hydrogen, it preferably is identical to $R_3$. When $R_5$ is not hydrogen, it preferably is identical to $R_3$ and $R_4$. When $R_2$ is disubstituted phenyl and/or $R_e$ is di- or trisubstituted phenyl or phenylalkyl, the substituents of the phenyl ring preferably are identical.

A group of compounds of formula I are the compounds of formula Ip

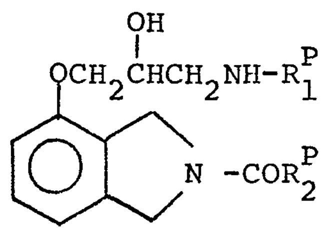

Ip

wherein

$R_1^P$ is alkyl of 3 to 7 carbon atoms or a group

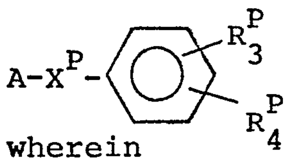

wherein

A is as defined above,

$X^P$ is a bond or an oxygen atom,

$R_3^P$ is hydrogen, alkoxy of 1 to 4 carbon atoms or hydroxy and

$R_4^P$ is hydrogen and, when $R_3^P$ is alkoxy of 1 to 4 carbon atoms, $R_4^P$ additionally may be alkoxy of 1 to 4 carbon atoms,

with the proviso that $X^P$ is separated from the nitrogen atom of the 3-aminopropoxy side chain by at least 2 carbon atoms, and

$R_2^P$ has the significance indicated above for $R_2$.

In accordance with the invention, a compound of the invention may be obtained by reacting a compound of formula F

$$\text{Het} -OCH_2-R_o \qquad F$$

wherein Het is a heterocyclic nucleus which comprises an N-carbonylisoindolinyl radical and the oxygen atom is attached directly to the 4 position of the isoindolinyl ring, and $R_o$ is a group capable of reacting with a primary or secondary amine to give a 2-amino-1-hydroxyethyl group, with a appropriate amine, and, where required, appropriately substituting the hydroxy group in the 2 position of the 3-aminopropoxy side chain.

Especially, compounds of formula I and physiologically acceptable hydrolyzable derivatives thereof may be obtained by ——

a process comprising reacting a compound of formula II

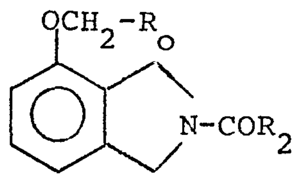

II

wherein $R_o$ and $R_2$ are as defined above, with a compound of formula III

$$R_1 - NH_2 \qquad III$$

wherein $R_1$ is as defined above, and, where required, appropriately esterifying the 2 position of the 3-amino-propoxy side chain in the resulting compound of formula I.

The amination process ________________________________ ____________ may be effected in conventional manner for the production of analogous 3-amino-2-hydroxypropoxyaryl compounds. For example $R_o$ may be a group of formula 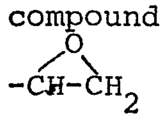 or a reactive derivative of this group, e.g. of formula $-CH(OH)-CH_2Y$, wherein Y is ________________

——— chlorine or bromine, or a group $R_y-SO_2-O$, wherein $R_y$ is phenyl, tolyl or lower alkyl. Y is especially chlorine. The reaction is preferably effected in an inert organic solvent, e.g. in an appropriate ether such as dioxane. Optionally an excess of the amine may be used as solvent. Alternatively the reaction may be effected in a fusion melt. Suitable reaction temperatures may be from about 20 to about 200°C, conveniently the reflux temperature of the reaction mixture when a solvent is present.

The optional substitution of the 2-hydroxy group in the side chain may be effected in conventional manner. For example, it may be esterified in manner known for the production of analogous esters of 3-amino-2-hydroxypropoxyaryl compounds. When $R_3$ is hydroxy, such esterification step is effected selectively in the 2 position of the 3-amino-propoxy side chain, conveniently under temporary protection of a such hydroxy group $R_3$ in the form of e.g. a benzyloxy group and subsequent selective splitting of the protecting group, e.g. by hydrogenation.

Free base forms of the compounds of the invention may be converted into salt forms in conventional manner and vice versa. Suitable acids for acid addition salt formation include maleic, malonic and fumaric acid. When $R_3$ is hydroxy, salts may be formed with strong bases, e.g. sodium hydroxide.

In the compounds of the invention, the carbon atom in 2 position of the 3-aminopropoxy side chain is asymmetrically substituted. The compounds may thus exist in the racemic form or in individual optical isomer form. The preferred optical isomer has the S configuration at the asymmetrically substituted carbon atom of the 3-aminopropoxy side chain.

Individual optical isomer forms may be obtained in conventional manner, for example by using optically active starting materials or by fractional crystallisation using optically active acids.

A compound of formula F forms part of the present invention, and may be obtained by introducing by O-alkylation a group $-O-CH_2R_o$ into a compound of formula Het-OH, wherein Het is as defined above.

In particular, a compound of formula II may be obtained by introducing by O-alkylation a group $-OCH_2-R_o$ into a compound of formula IV

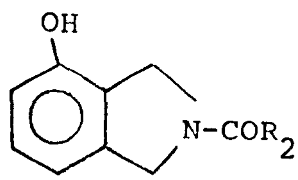

IV

wherein $R_2$ is as defined above. The compounds of formula IV are preferably reacted in anionic form.

A compound of formula Het-OH, wherein Het is as defined above, forms part of the present invention and may be obtained by introducing a carbonyl group into the 2-position of the corresponding isoindolinol.

Similarly, a compound of formula IV may be obtained by introducing a group $-COR_2$ in the 2 position of 4-isoindolinol.

Insofar as the preparation of any particular starting material is not particularly described, this may be effected in conventional manner.

In the following examples all temperatures are in degrees Centigrade and are uncorrected.

Example 1: 1-(2-acetylisoindolin-4-yloxy)-3-(3,4-dimethoxyphenethylamino)-2-propanol

2,5 g 2-acetyl-4-(2,3-epoxypropoxy)-isoindoline and 1 ml 3,4-dimethoxyphenethylamine dissolved in 50 ml dioxane are kept at 130° in an autoclave for 20 hours. The solvent is then evaporated, the residue dissolved in methylene chloride and extracted with 1N tartaric acid. The aqueous phase is then separated, made alkaline with dilute sodium hydroxide solution, and extracted with methylene chloride. The title compound is formed (M.P. of the hydrogen maleate form 177-178° - from methanol/ether).

The starting material 2-acetyl-4-(2,3-epoxypropoxy)-isoindoline is obtained as follows:

a) Reaction of acetyl chloride with isoindolin-4-ol hydrochloride in pyridine at 25° yields 2-acetyl-isoindolin-4-ol (M.P. 256-258° [dec.] - from methanol/ether).

b) Reaction at 100° of 2-acetylisoindolin-4-ol with epichlorhydrin in excess and in presence of catalytic amounts of piperidine yields 2-acetyl-4-(2,3-epoxypropoxy)isoindoline (M.P. 100-102° - from methylene chloride/ether).

Example 2: 1-(2-acetylisoindolin-4-yloxy)-3-(3,4-dimethoxyphenethylamino)-2-propanol benzoate

2.0 g of benzoic acid and 1.0 g of 1-(2-acetyl-isoindolin-4-yloxy)-3-(3,4-dimethoxyphenethylamino)-2-propanol in 10 ml of chloroform are reacted dropwise at 0° with 0.65 g of benzoic anhydride in 10 ml of chloroform. After 1 hour at 50°, an excess of a 2N sodium hydroxide solution is added and the reaction mixture is extracted thrice with methylene chloride, the combined organic phases are dried over $MgSO_4$, filtered and concentrated. The title compound is formed (M.P. of the hydrogen fumarate form 153-155° - from methanol/ether).

From the appropriate compound of formula II, wherein $R_o$ is $-CH(OH)-CH_2Cl$, and the appropriate compound of formula III the following compounds of formula I may be obtained in analogous manner to Example 1:

| Ex.No | $R_1$ | $R_2$ | M.P. |
|---|---|---|---|
| 3 | isopropyl | phenyl | ch 206-207° |
| 4 | isopropyl | hydrogen | hfu 156-158° |
| 5 | 2-phenoxyethyl | hydrogen | hfu 176-178° |
| 6 | 2-methyl-4-phenylbut-2-yl | hydrogen | bfu 213-214° |
| 7 | 3-(4-hydroxyphenyl)-2-methylprop-2-yl | hydrogen | bfu 198-200° |
| 8 | tert-butyl | methyl | hfu 199-201° |
| 9 | isopropyl | methyl | bfu 166-170° |
| 10 | isopropyl | ethoxy | b 127-128° |
| 11 | tert-butyl | hydrogen | bfu 189-191° |
| 12 | 3,4-dimethoxyphenethyl | hydrogen | b 148-150° |
| 13 | 3,4-methylenedioxyphenethyl | hydrogen | hfu 153-155° |

b = free form

bfu = bis[base]fumarate form

ch = hydrochloride form

hfu = hydrogen fumarate form

From the appropriate compound of formula I, the following compound of formula E may be obtained in analogous manner to Example 2:

| Ex.No. | $R_1$ | $R_2$ | $R_e$ | M.P. |
|---|---|---|---|---|
| 14 | 3,4-dimethoxy-phenethyl | methyl | tert-butyl | oil |

The compounds of the invention exhibit pharmacological activity.

In particular, the compounds possess β-adrenergic blocking activity, as indicated by standard tests. For example, in the isolated, spontaneously-beating guinea pig atrium (method of K. Saameli, Helv.Physiol. Acta 25 [1967] CR 219-CR 221) inhibition of the positive inotropic effect of adrenaline is observed at a bath concentration of about $10^{-5}$ to about $10^{-3}$ mg/l.

The compounds are therefore indicated for use as β-adrenergic blocking agents, e.g. for the prophylaxis and treatment of hypertension, of coronary diseases, such as Angina pectoris, of conditions resulting from sympathetic overstimulation, such as nervous heart ailments, of myocardial infarct, for interval migraine treatment, and for the treatment of glaucoma, thyreotoxicosis and arrhythmias.

The compounds also exhibit α-adrenergic blocking activity, as indicated by standard tests. For example, the inhibition of α-adrenoceptors may be observed in isolated spiral strips of the Vena

femoralis of dogs (E. Müller-Schweinitzer and E. Stürmer, <u>Br.J. Pharmacol</u>. [1974] 51, 441-446) at a bath concentration of from about $10^{-7}$ M to about $10^{-5}$ M.

The compounds are therefore indicated for use as α-adrenergic blocking agents, e.g. for the prophylaxis and treatment of disorders related to a paralysis of intestine motility, such as paralytic ileus.

It will be appreciated that it may be necessary to convert a compound having a substituted hydroxy group in the 2 position of the 3-aminopropoxy side chain to the corresponding free hydroxy compound prior to carrying out the tests indicated above for showing α- and β-adrenergic blocking activity.

An indicated daily dose is from about 5 mg to about 500 mg, conveniently given in divided doses 2 to 4 times a day in unit dosage form containing from about 1.25 mg to about 250 mg, or in sustained release form.

The compounds wherein a group -A-X- 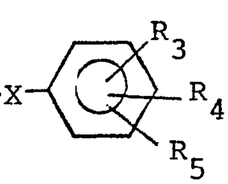 is attached to the nitrogen atom of the 3-aminopropoxy side chain are especially interesting as cardioselective β-blocking agents.

The compounds of Examples 1 and 12 exhibit particularly interesting activity as β-blockers.

In general, the 2(S) optical isomers of the compounds are more active than the 2 (R) optical isomers as β-blocking agents.

The compounds may be administered in pharmaceutically acceptable salt form. Such salt forms exhibit the same order of activity as the free forms and are readily prepared in conventional manner. The present invention also provides a pharmaceutical composition comprising a compound of the invention, in free form or in pharmaceutically acceptable salt form, in association with a pharmaceutical carrier or diluent. Such compositions may be in the form of, for example, a solution or a tablet.

**What we claim is:-**

1. An adrenergic blocking agent of the type having an 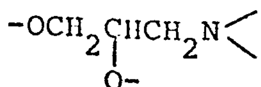 side chain attached through the 1-oxgen atom thereof directly to a heterocyclic nucleus, in free form or in salt form, characterized in that the heterocyclic nucleus comprises an N-carbonylisoindolinyl radical and the 1-oxygen atom of the side chain is attached directly to the 4 position of the isoindolinyl ring.

2. A compound having the formula I

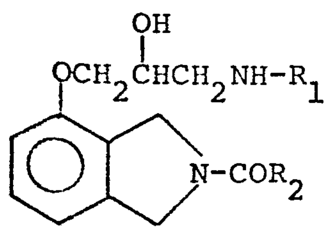

I

wherein

$R_1$ is alkyl of 3 to 7 carbon atoms or a group

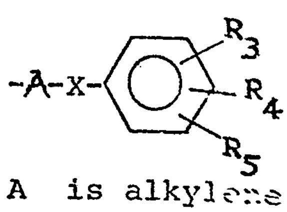

wherein

A is alkylene of 2 to 5 carbon atoms,

X is a bond, an oxygen or a sulfur atom, either

$R_3$ is hydrogen, halogen of atomic number of from 9 to 35, alkyl of 1 to 4 carbon atoms, alkoxy of 1 to 4 carbon atoms or hydroxy, and

$R_4$ is hydrogen and, when $R_3$ is halogen of atomic number of from 9 to 35, $R_4$ additionally may be halogen of atomic number of from 9 to 35 and, when $R_3$ is alkoxy of 1 to 4 carbon atoms, $R_4$ additionally may be alkoxy of 1 to 4 carbon atoms, or

$R_3$ and $R_4$ together are methylenedioxy or ethylenedioxy, in the 2,3 or the 3,4 position,

$R_5$ is hydrogen and, when $R_3$ and $R_4$ both are alkoxy of 1 to 4 carbon atoms, $R_5$ additionally may be alkoxy of 1 to 4 carbon atoms, with the proviso that X is separated by at least 2 carbon atoms from the nitrogen atom of the 3-aminopropoxy side chain, and

$R_2$ is hydrogen, alkyl of 1 to 4 carbon atoms, alkoxy of 1 to 4 carbon atoms or phenyl unsubstituted or monosubstituted or independently disubstituted by alkyl of 1 to 4 carbon atoms, alkoxy of 1 to 4 carbon atoms, or halogen of atomic number of from 9 to 35,

and physiologically acceptable hydrolyzable derivatives thereof having the hydroxy group in the 2 position of the 3-aminopropoxy side chain in esterified form,

in free form or in salt form.

3. A compound of claim 2 of formula Ip,

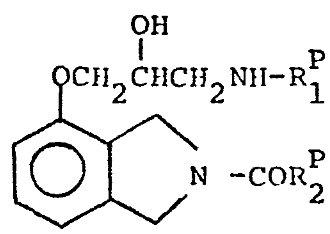

Ip

wherein

$R_1^P$ is alkyl of 3 to 7 carbon atoms or a group

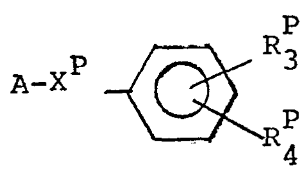

wherein

A is as defined in claim 2,

$X^P$ is a bond or an oxygen atom,

$R_3^P$ is hydrogen, alkoxy of 1 to 4 carbon atoms or hydroxy and

$R_4^P$ is hydrogen and, when $R_3^P$ is alkoxy of 1 to 4 carbon atoms, $R_4^P$ additionally may be alkoxy of 1 to 4 carbon atoms,

with the proviso that $X^P$ is separated from the nitrogen atom of the 3-aminopropoxy chain by at least 2 carbon atoms, and

$R_2^P$ has the significance indicated in claim 2 for $R_2$.

4. The compound of any one of claims 1 to 3, which is 1-(2-acetylisoindolin-4-yloxy)-3-(3,4-dimethoxyphenethylamino)-2-propanol.

5. The compound of claim 2, wherein
either $R_1$ is isopropyl and $R_2$ is phenyl,
or $R_1$ is isopropyl and $R_2$ is hydrogen,
or $R_1$ is 2-phenoxyethyl and $R_2$ is hydrogen,
or $R_1$ is 2-methyl-4-phenylbut-2-yl and $R_2$ is hydrogen,
or $R_1$ is 3-(4-hydroxyphenyl)-2-methylprop-2-yl and $R_2$ is hydrogen,
or $R_1$ is tert-butyl and $R_2$ is methyl,
or $R_1$ is isopropyl and $R_2$ is methyl,
or $R_1$ is isopropyl and $R_2$ is ethoxy,
or $R_1$ is tert-butyl and $R_2$ is hydrogen,
or $R_1$ is 3,4-dimethoxyphenethyl and $R_2$ is hydrogen,
or $R_1$ is 3,4-methylenedioxyphenethyl and $R_2$ is hydrogen.

6. The compound of claim 2, wherein $R_1$ is 3,4-dimethoxyphenethyl, $R_2$ is methyl and the hydroxy group in the 2 position of the 3-aminopropoxy side chain is esterified with either a benzoyl or a tert-butyl group.

7. A process for the production of a compound of claim 1, which comprises reacting a compound of formula F

$$Het-OCH_2-R_o \qquad F$$

wherein Het is a heterocyclic nucleus which comprises an N-carbonylisoindolinyl radical and the oxygen atom is attached directly to the 4 position of the isoindolinyl ring, and $R_o$ is a group capable of reacting with a primary or secondary amine to give a 2-amino-1-hydroxyethyl group, with an appropriate amine, and, where required, appropriately substituting the hydroxy group in the 2 position of the 3-aminopropoxy side chain.

8. A process for the production of a compound of claim 2, in free form or in salt form, which comprises reacting a compound of formula II

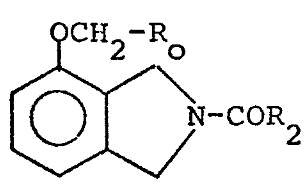

II

wherein $R_2$ is as defined in claim 2 and $R_o$ is as defined in claim 7, with a compound of formula III,

$$R_1 - NH_2 \qquad III$$

wherein $R_1$ is as defined in claim 2, and, where required, appropriately esterifying the 2 position of the 3-amino-propoxy side chain in the resulting compound of formula I.

9. A pharmaceutical composition comprising a compound of any one of claims 1 to 6 in free form or in pharmaceutically acceptable salt form, in association with a pharmaceutical carrier or diluent.

10. A compound of formula Het-$OCH_2$-$R_o$ or Het-OH, wherein Het and $R_o$ are as defined in claim 7.

11. A compound of formula II,

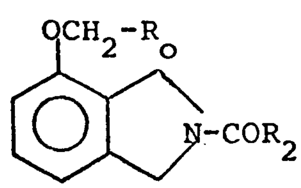

II

wherein $R_2$ is as defined in claim 2 and $R_o$ is as defined in claim 7, or a compound of formula IV,

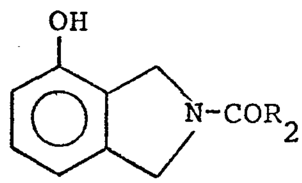

IV

wherein $R_2$ is as defined in claim 2.

European Patent Office

# EUROPEAN SEARCH REPORT

0002792
Application number

EP 78 101 777.7

| DOCUMENTS CONSIDERED TO BE RELEVANT | | | CLASSIFICATION OF THE APPLICATION (Int. Cl.²) |
|---|---|---|---|
| Category | Citation of document with indication, where appropriate, of relevant passages | Relevant to claim | |
| | US - A - 3 976 779 (BOEHRINGER MANNHEIM)<br>* column 1, lines 38 to 68, column 2, lines 1 to 8, claim 9 *<br>-- | 7-9 | C 07 D 209/44<br>A 61 K 31/40<br>C 07 D 405/12 |
| | GB - A - 1 343 834 (SANDOZ)<br>* page 1, column 1, lines 51 to 64 *<br>-- | 8 | |
| | US - A - 4 016 283 (CIBA-GEIGY)<br>* claim 1 *<br>-- | 9 | TECHNICAL FIELDS SEARCHED (Int.Cl.²)<br>A 61 K 31/40<br>C 07 D 209/44<br>C 07 D 405/12 |
| A | US - A - 3 471 515 (SANDOZ)<br>* abstract *<br>-- | | |
| A | G. EHRHART et al " Arzneimittel", Vol. 2, 1972, Verlag Chemie, Weinheim pages 177 to 181<br>---- | | CATEGORY OF CITED DOCUMENTS<br>X: particularly relevant<br>A: technological background<br>O: non-written disclosure<br>P: intermediate document<br>T: theory or principle underlying the invention<br>E: conflicting application<br>D: document cited in the application<br>L: citation for other reasons |
| X | The present search report has been drawn up for all claims | | &: member of the same patent family, corresponding document |

| Place of search | Date of completion of the search | Examiner |
|---|---|---|
| Berlin | 06-04-1979 | FROELICH |

EPO Form 1503.1 06.78